# EUROPEAN PATENT SPECIFICATION

(11) **EP 3 824 875 B1**
(45) Date of publication and mention of the grant of the patent: **11.10.2023**
(21) Application number: 18923974.2
(22) Date of filing: 17.12.2018
(51) Int. Cl.: A61K 8/02, A61K 8/25, A61K 8/64, A61K 8/98, A61K 47/69, A61Q 19/08, A61Q 19/00

(54) **MICRONEEDLE COATED WITH DRUG AND MANUFACTURING METHOD FOR SAME**
MIT ARZNEIMITTEL BESCHICHTETE MIKRONADEL UND HERSTELLUNGSVERFAHREN DAFÜR
MICRO-AIGUILLE REVÊTUE D'UN MÉDICAMENT ET SON PROCÉDÉ DE FABRICATION

(30) Priority: 29.06.2018 KR 20180076060
(43) Date of publication of application: 26.05.2021
(73) Proprietor: SR Biotek Inc., Gyeonggi-do 13229 (KR)
(72) Inventor: SONG, Byung Ho, Seongnam-si Gyeonggi-do 13523 (KR); JEONG, Kwang Taek, Paju-si Gyeonggi-do 10813 (KR); JI, Min Jae, Seongnam-si Gyeonggi-do 13565 (KR); NAM, Da Yeong, Seongnam-si Gyeonggi-do 13148 (KR); EOM, Hye Seon, Seoul 05530 (KR); KIM, Ji Young, Seongnam-si Gyeonggi-do 13250 (KR)
(74) Representative: Lahrtz, Fritz
(86) International application number: PCT/KR2018/016032
(87) International publication number: WO 2020/004745

(56) References cited:
- WO-A1-2017/186046
- KR-A- 20160 064 410
- KR-A- 20180 011 653
- KR-B1- 100 937 389
- KR-B1- 101 728 125
- MCINNES et al.: "Silicon-polymer hybrid materials for drug deliver", Future Med. Chem, vol. 1, no. 6, 2009, pages 1051-1074, XP055673064,

## Description

### Technical Field

The present invention relates to a drug-coated microneedle and a method of manufacturing the same, and more particularly to a drug-coated microneedle that delivers a drug by physically piercing the stratum corneum of the skin and a method of manufacturing the same.

### Background Art

The skin, which protects the human body from the external environment and is the most important biochemical and physical tissue, is broadly divided into the epidermis, dermis and hypodermis. The stratum corneum, which is the outermost layer of the skin, has a function of regulating moisture evaporation and absorption by the skin and a barrier function against penetration of chemicals, toxic substances, bacteria and the like. As for the stratum corneum, keratinocytes produced in the basal layer take about 14 days to reach the granular layer. In the granular layer, when keratinization is initiated by the action of keratohyalin, the body fluid in the cells escapes and the cells are keratinized in a flattened state. Millions of keratinocytes are released from the skin every day, and new keratinocytes are produced to replace the same.

One of the signs of skin aging is that the skin loses vitality and elasticity with an increase in the cell regeneration cycle due to decreased ability to shed dead cells. Therefore, many attempts are being made to remove the epidermal layer through a skin-peeling technique to thus stimulate cell regeneration in the basal layer in order to restore skin elasticity.

Cosmetics for exfoliation developed to date may be roughly classified into three types of exfoliation methods. The first is a method of finely cutting the epidermis by physical action such as skin friction or a laser, the second is a chemical method that uses chemical components such as AHA (alpha-hydroxy acid), BHA (beta-hydroxy acid), urea and the like, which have a keratolysis effect, and the third is a method of removing keratin using proteolytic enzymes having a function of exfoliation. In these peeling methods, the skin is first peeled off, and then the regenerative capacity of the skin itself is induced.

However, in the case of physical abrasion, the skin swells red due to significant irritation when applied to the skin, or intense pain occurs, so an anesthetic is used during the procedure, and thus there is a problem in that it is difficult to use at home. Also, in the case of chemical peeling, it is difficult to use at home, and there is a problem in that pigmentation-related abnormal skin discoloration, infection or complications such as permanent sequelae such as scars may occur. Moreover, peeling using proteolytic enzymes is expensive, and is also unsuitable for use at home due to problems related to the storage and use thereof.

With the goal of solving the above problems, research is ongoing into the application of sponge spicules to cosmetic compositions. Korean Patent No. 0937389 discloses a method of preparing a skin care cosmetic composition including extracting microneedles from sponges using a hydrogen peroxide solution, increasing the porosity of the extracted microneedles, coating the microneedles having the increased porosity with a mixed solution of a stem-cell-derived material and a skin active ingredient through immersion, and freeze-drying the mixed solution in which the microneedles are immersed, and Korean Patent No. 1810231 discloses a cosmetic composition for reducing acne and atopy, containing, as active ingredients, a complex extract composed of a propolis extract, a royal jelly extract, a golden extract and a red ginseng extract, and a microneedle powder. Korean Patent No. 1854446 discloses a cosmetic composition containing a *Sageretia theezans* extract and a microneedle.

WO2017186046 A1 discloses the use of sponge or sponge spicules as skin penetration enhancers for cosmetics. KR101728125 B1 discloses making cosmetic needles from sponge, spray-dried with acetylhexapeptide 8 and palmitoylpentapeptide.

However, when the microneedle is immersion-coated or simply coated with the active ingredient drug, the immersion-coating rate may be low depending on the type of drug, or the drug may not be released after penetration into the skin, so the effects thereof are insignificant.

Therefore, the present inventors have endeavored to solve the problems encountered in the related art, and thus ascertained that, when using a microneedle having a drug attached thereto through disulfide bonding, the drug may be released through a redox reaction after penetration into the skin, making it possible to effectively deliver even a drug having excellent functionality but low skin permeability, thus culminating in the present invention.

### Disclosure

### Technical Problem

An objective of the present invention is to provide a microneedle having excellent drug delivery capability and a method of manufacturing the same.

Another objective of the present invention is to provide a cosmetic composition, which has excellent drug delivery capability and is easy to use, and is thus usable at home.

### Technical Solution

In order to accomplish the above objectives, the present invention provides a method of manufacturing a drug-coated microneedle, which is represented by Chemical Formula 1 and in which a drug is released through a redox reaction after penetration into the skin, including: (a) manufacturing a silica- (SiO₂)-containing microneedle having a sulfhydryl (-SH) functional group by modifying the surface of a silica-(SiO₂)-containing microneedle; (b) manufacturing a drug having a sulfhydryl (-SH) functional group by binding a drug with a linker having a sulfhydryl (-SH) functional group; and (c) subjecting the silica-(SiO₂)-containing microneedle having the sulfhydryl (-SH) functional group and the drug having the sulfhydryl (-SH) functional group to an oxidation reaction.

In the present invention, the modifying the surface of the silica-(SiO₂)-containing microneedle may be performed by subjecting the silica-(SiO₂)-containing microneedle to stirring with any one selected from the group consisting of (3-mercaptopropyl)methyldimethoxysilane, (3-mercaptopropyl)trimethoxysilane, thioglycol ic acid, 3-mercaptopropionic acid, 11-mercaptoundecanoic acid, and 3,3'-dithiodipropionic acid.

In the present invention, the oxidation reaction of the silica-(SiO₂)-containing microneedle having the sulfhydryl (-SH) functional group and the drug having the sulfhydryl (-SH) functional group may be performed using at least one oxidant selected from the group consisting of iodine, potassium phosphate, 2,3-dichloro-5, 6-dicyano-p-benzoquinone (DDQ), dehydroascorbic acid, hydrogen peroxide (H₂O₂), and oxygen (O₂).

In addition, the present invention provides a drug-coated microneedle, which is represented by Chemical Formula 1 below and in which a drug is released through a redox reaction after penetration into the skin.

[Chemical Formula 1] MN-S-S-D

In Chemical Formula 1, MN is a silica-(SiO₂)-containing microneedle, S-S is a disulfide bond, and D is a drug.

In the present invention, the silica-(SiO₂)-containing microneedle may be an acicular spicule derived from a sponge.

In the present invention, the microneedle represented by Chemical Formula 1 may be manufactured by attaching a silica-(SiO₂)-containing microneedle having a sulfhydryl (-SH) functional group and a drug having a sulfhydryl (-SH) functional group to each other.

In the present invention, the silica-(SiO₂)-containing microneedle may include the sulfhydryl (-SH) functional group in an amount of 0.8 × 10⁻⁶ mol/g to 10 × 10⁻⁶ mol/g.

In the present invention, the drug having the sulfhydryl (-SH) functional group may be at least one selected from the group consisting of Chemical Formulas 2 to 8 below.

[Chemical Formula 2]: Lys-His-Gly-X

[Chemical Formula 3]: Gly-His-Lys-X

[Chemical Formula 4]: Ser-Lys-Thr-Thr-Lys-X

[Chemical Formula 5]: Lys-Thr-Thr-Lys-Ser-X

[Chemical Formula 6]: Arg-Arg-Gln-Met-Glu-Glu-X

[Chemical Formula 7]: Glu-Glu-Met-Gln-Arg-Arg-X

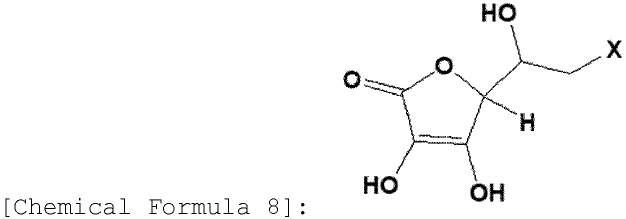

In Chemical Formulas 2 to 8, X is at least one selected from the group consisting of thioglycolic acid, 3-mercaptopropionic acid, 11-mercaptoundecanoic acid, and cysteine.

In addition, the present invention provides a cosmetic composition including the above microneedle.

In the present invention, the cosmetic may be selected from the group consisting of a softening lotion, nourishing lotion, nourishing cream, massage cream, essence, ampoule, gel, eye cream, cleansing cream, cleansing foam, cleansing water, pack, spray, and powder.

### Advantageous Effects

According to the present invention, a drug-coated microneedle is capable of effectively delivering a drug having excellent functionality but low skin permeability, and is thus useful as a material for functional cosmetics for whitening, wrinkle reduction, inflammation reduction and the like.

### Brief Description of Drawings

FIG. 1 is SEM and microscope images of a silica-(SiO₂)-containing microneedle purified according to an embodiment of the present invention;
FIG. 2 is graphs showing the results of measurement of the molar number of a sulfhydryl (-SH) functional group per g of a microneedle having a sulfhydryl (-SH) functional group manufactured according to an embodiment of the present invention;
FIG. 3 is a graph showing the results of evaluation of cytotoxicity of a drug having a sulfhydryl (-SH) functional group manufactured according to an embodiment of the present invention;
FIG. 4 is graphs showing the results of evaluation of collagen production capability of the drug having the sulfhydryl (-SH) functional group manufactured according to an embodiment of the present invention;
FIG. 5 is a graph showing the results of evaluation of collagen production capability of the drug detached from the microneedle depending on the type of sulfhydryl (-SH) functional group;
FIG. 6 is a graph showing the results of evaluation of collagen production capability of the drug depending on the amount of GSH (glutathione); and
FIG. 7 is microscope images showing the wrinkle reduction effect of a cream manufactured according to an embodiment of the present invention.

### Best Mode

When a drug having excellent functionality but low skin permeability is attached to a microneedle through disulfide bonding, the drug penetrates into the stratum corneum of the skin along with the microneedle and is then released through a redox reaction, so the present invention is intended to confirm that the drug can be effectively delivered.

In the present invention, a drug-coated microneedle is manufactured by modifying the surface of a microneedle derived from an animal sponge to afford a silica-(SiO₂)-containing microneedle having a sulfhydryl (-SH) functional group, binding a drug with a linker having a sulfhydryl (-SH) functional group to afford a drug having a sulfhydryl (-SH) functional group, and performing an oxidation reaction therebetween. Based on the results of evaluation of cytotoxicity and collagen production capability of the drug-coated microneedle thus manufactured, it is confirmed that the drug-coated microneedle has no cytotoxicity but has superior collagen production effects.

Therefore, an aspect of the present invention pertains to a method of manufacturing a drug-coated microneedle, which is represented by Chemical Formula 1 below and in which a drug is released through a redox reaction after penetration into the skin, including: (a) manufacturing a silica-(SiO₂)-containing microneedle having a sulfhydryl (-SH) functional group by modifying the surface of a silica-(SiO₂)-containing microneedle; (b) manufacturing a drug having a sulfhydryl (-SH) functional group by binding a drug with a linker having a sulfhydryl (-SH) functional group; and (c) subjecting the silica-(SiO₂)-containing microneedle having the sulfhydryl (-SH) functional group and the drug having the sulfhydryl (-SH) functional group to an oxidation reaction.

[Chemical Formula 1] MN-S-S-D

In Chemical Formula 1, MN is a silica-(SiO₂)-containing microneedle, S-S is a disulfide bond, and D is a drug.

In the present invention, the silica-(SiO₂)-containing microneedle may be an acicular spicule derived from a sponge. Examples of the sponge may include, but are not limited to, *Spongilla lacustris, Spongilla fragilis, Ephydatia fluviatilis,* and the like.

The size of the acicular spicule is not particularly limited, and an acicular spicule having a length of about 100 to 300 um and a width of about 10 to 30 um may be used.

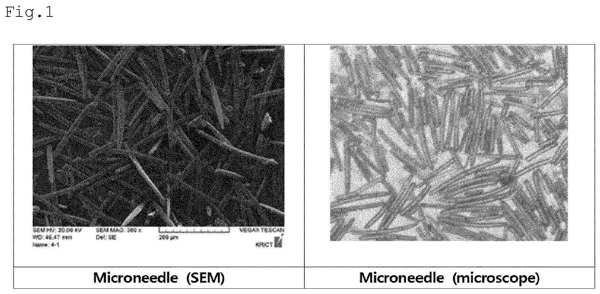

The silica- (SiO₂)-containing microneedle that is used is preferably one purified in a manner in which an acicular spicule powder derived from a sponge is sequentially added with sulfuric acid and sodium hydroxide, stirred, filtered, and washed. FIG. 1 is SEM and microscope images of the silica- (SiO₂)-containing microneedle purified according to an embodiment of the present invention.

In order to penetrate into the skin, the drug, such as a peptide, which alone has difficulty penetrating into the skin, has to be attached to the microneedle. In order to attach the drug to the microneedle through disulfide bonding, a sulfhydryl (-SH) functional group has to be introduced to each of the drug and the microneedle.

First, in the present invention, a silica-(SiO₂)-containing microneedle having a sulfhydryl (-SH) functional group is manufactured by modifying the surface of a silica-(SiO₂)-containing microneedle.

Here, the modifying the surface of the silica-(SiO₂)-containing microneedle is performed by subjecting the silica-(SiO₂)-containing microneedle to stirring with any one selected from the group consisting of (3-mercaptopropyl)methyldimethoxysilane, (3-mercaptopropyl)trimethoxysilane, thioglycolic acid, 3-mercaptopropionic acid, 11-mercaptoundecanoic acid, and 3,3'-dithiodipropionic acid.

Although varying depending on the shape of the microneedle, the type of mercaptosilane for surface modification, and the reaction conditions, the silica-(SiO₂)-containing microneedle thus manufactured may include the sulfhydryl (-SH) functional group in an amount of 0.8 × 10⁻⁶ mol/g to 10 × 10⁻⁶ mol/g, and preferably in an amount exceeding 2.0 × 10⁻⁶ mol/g, taking into consideration the surface area of the microneedle.

In the present invention, a drug having a sulfhydryl (-SH) functional group is manufactured by binding a drug with a linker having a sulfhydryl (-SH) functional group.

As the drug, any material may be used without particular limitation, so long as it penetrates into the skin and is capable of increasing skin improvement effects, such as skin-soothing effects, vitality enhancement effects, antioxidant effects, whitening effects, moisturizing effects, skin regeneration effects, anti-aging effects, anti-inflammatory effects, collagen synthesis promotion effects, and the like. Not only a synthetic compound but also a peptide may be used.

Examples of the linker having the sulfhydryl (-SH) functional group may include, but are not limited to, thioglycolic acid, 3-mercaptopropionic acid, 11-mercaptoundecanoic acid, cysteine, and the like.

When the drug is a peptide, the use of cysteine as the linker may cause the properties thereof to vary depending on the amino acid sequence and structure, and thus the use of thioglycolic acid, 3-mercaptopropionic acid or 11-mercaptoundecanoic acid is preferable, with the use of thioglycolic acid, having a low molecular weight, being most preferable.

In the present invention, examples of the drug having the sulfhydryl (-SH) functional group manufactured by binding the peptide or compound drug with the linker having the sulfhydryl (-SH) functional group may include, but are not limited to, Chemical Formulas 2 to 8 below.

[Chemical Formula 2]: Lys-His-Gly-X

[Chemical Formula 3]: Gly-His-Lys-X

[Chemical Formula 4]: Ser-Lys-Thr-Thr-Lys-X

[Chemical Formula 5]: Lys-Thr-Thr-Lys-Ser-X

[Chemical Formula 6]: Arg-Arg-Gln-Met-Glu-Glu-X

[Chemical Formula 7]: Glu-Glu-Met-Gln-Arg-Arg-X

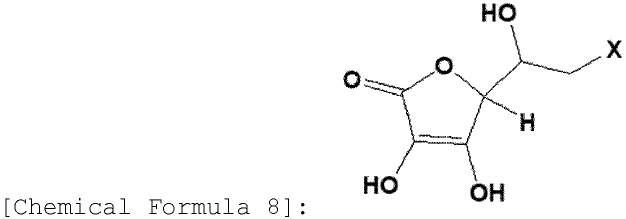

In Chemical Formulas 2 to 8, X is at least one selected from the group consisting of thioglycolic acid, 3-mercaptopropionic acid, 11-mercaptoundecanoic acid, and cysteine.

In the present invention, a drug-coated microneedle is manufactured by subjecting the silica-(SiO₂)-containing microneedle having the sulfhydryl (-SH) functional group and the drug having the sulfhydryl (-SH) functional group to an oxidation reaction.

As the oxidant for the oxidation reaction, any material may be used without particular limitation, so long as it is able to attach the silica-(SiO₂)-containing microneedle having the sulfhydryl (-SH) functional group and the drug having the sulfhydryl (-SH) functional group to each other through oxidation. Examples of the oxidant may include iodine, potassium phosphate, 2,3-dichloro-5,6-dicyano-p-benzoquinone (DDQ), dehydroascorbic acid, hydrogen peroxide (H₂O₂), oxygen (O₂), and the like.

In the present invention, a drug-coated microneedle capable of releasing a drug through a redox reaction after penetration into the skin is manufactured using the above method.

Therefore, another aspect of the present invention pertains to a drug-coated microneedle, which is represented by Chemical Formula 1 below and in which a drug is released through a redox reaction after penetration into the skin.

[Chemical Formula 1] MN-S-S-D

In Chemical Formula 1, MN is a silica-(SiO₂)-containing microneedle, S-S is a disulfide bond, and D is a drug.

In addition, the present invention pertains to a cosmetic composition including the drug-coated microneedle.

The amount of the drug-coated microneedle may vary depending on the type of cosmetic, and may be 0.1 to 10.0 wt% based on the total weight of the cosmetic composition.

Examples of the cosmetics may include, but are not limited to, a softening lotion, nourishing lotion, nourishing cream, massage cream, essence, ampoule, gel, eye cream, cleansing cream, cleansing foam, cleansing water, pack, spray, powder, and the like.

In the cosmetic composition, other ingredients may be appropriately combined within a range that does not impair the purpose according to the present invention depending on the type or purpose of use of the formulation, in addition to the drug-coated microneedle.

Moreover, depending on the quality or function of the final product, the cosmetic composition may further include materials typically used in the art, such as fatty materials, organic solvents, solubilizers, thickeners, gelling agents, emollients, antioxidants, suspension agents, stabilizers, foaming agents, fragrances, surfactants, water, ionic or nonionic emulsifiers, fillers, metal-ion-sequestering agents, chelating agents, preservatives, blocking agents, wetting agents, essential oils, dyes, pigments, hydrophilic or lipophilic activating agents, and adjuvants commonly used in the cosmetic or dermatological field, such as any other ingredients commonly used in cosmetic compositions.

Here, it is preferable that the adjuvants and the mixing ratio thereof be appropriately selected so as not to affect the desirable properties of the cosmetic composition according to the present invention.

### Mode for Invention

A better understanding of the present invention will be given through the following examples. These examples are merely set forth to illustrate the present invention, and are not to be construed as limiting the scope of the present invention, as will be apparent to those skilled in the art.

### Example 1: Manufacture of drug-coated microneedle

### 1-1: Purification of silica- (SiO₂) -containing microneedle

An acicular spicule (289 g) derived from *Spongilla fragilis* Leidy and sulfuric acid (80 g) were placed in a reactor, stirred for 1 hr, added with water, further stirred, and filtered. The acicular spicule was washed with water, further stirred for 1 to 2 hr, added with NaOH and HNO₃, stirred, adjusted to a pH of 6 to 8, and washed with water. Finally, the acicular spicule was washed with ethanol, filtered, and dried.

### 1-2: Surface modification of silica-(SiO₂)-containing microneedle

In order to introduce a sulfhydryl (-SH) functional group to the purified acicular spicule (silica-(SiO₂)-containing microneedle), hydrolysis and dealkoxylation were performed.

### 1-2-1: Surface modification using hydrolysis

As shown in [Scheme 1] below, 10 g of the purified microneedle, 0.1 to 3.0 ml of (3-mercaptopropyl)trimethoxysilane (MPTMS), and 100 g of a mixed water/ethanol solution (50% ethanol) were placed in a reactor and then allowed to react at room temperature for 4 hr. The resulting reaction product was washed and then dried under reduced pressure at 70°C for 4 hr, thus obtaining a silica-(SiO₂)-containing microneedle having a sulfhydryl (-SH) functional group (No. 1).

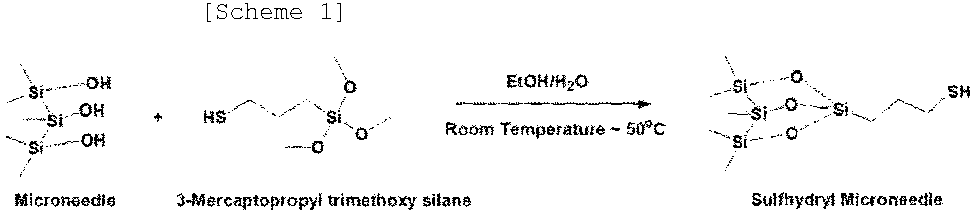

### 1-2-2 to 1-2-4: Surface modification using hydrolysis

The reaction was carried out under the conditions of Example 1-2-1, with the exception that the reaction time was changed to 8, 16 and 24 hr. After the reaction, the resulting reaction product was dried under reduced pressure at 70°C for 4 hr, thus obtaining silica-(SiO₂)-containing microneedles having sulfhydryl (-SH) functional groups (Nos. 2 to 4) .

### 1-2-5 and 1-2-6: Surface modification using hydrolysis

The reaction was carried out under the conditions of Example 1-2-1, with the exception that the reaction temperature was adjusted to 40°C and 60°C and the reaction time was changed to 24 hr. After the reaction, the resulting reaction product was dried under reduced pressure at 70°C for 4 hr, thus obtaining silica-(SiO₂)-containing microneedles having sulfhydryl (-SH) functional groups (Nos. 5 and 6) .

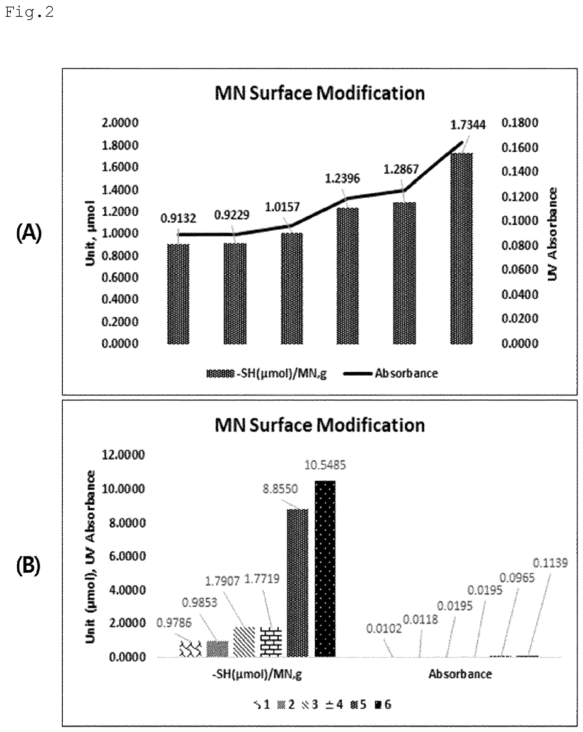

The molar number of the sulfhydryl (-SH) functional group per g of the silica-(SiO₂)-containing microneedle having the sulfhydryl (-SH) functional group obtained as described above was measured using a spectrophotometer (Agilent 8453 UV Spectrophotometer) through an Ellman assay. The results thereof are shown in Table 1 below and in FIG. 2.

**[Table 1]**

| No. | Abs. (412) | -SH molar concentration (M) | -SH molar number (mol) | -SH molar number (µmol) | SH molar number for 1 g of MN (µmol) |
|---|---|---|---|---|---|
| 1 | 0.08928 | 6.3095.E-06 | 1.3881.E-08 | 0.0139 | 0.9132 |
| 2 | 0.08963 | 6.3343.E-06 | 1.3935.E-08 | 0.0139 | 0.9229 |
| 3 | 0.09669 | 6.8332.E-06 | 1.5033.E-08 | 0.0150 | 1.0157 |
| 4 | 0.11959 | 8.4516.E-06 | 1.8593.E-08 | 0.0186 | 1.2396 |
| 5 | 0.12579 | 8.8898.E-06 | 1.9557.E-08 | 0.0196 | 1.2867 |
| 6 | 0.16510 | 1.1668.E-05 | 2.5669.E-08 | 0.0257 | 1.7344 |

As is apparent from Table 1 and FIG. 2, the -SH molar number for 1 g of MN was determined to be 0.9132 to 1.7344 pmol/g depending on the reaction temperature and the reaction time.

### 1-2-7: Surface modification using dealkoxylation

As shown in [Scheme 2] below, 5.02 g of the purified microneedle, 0.1 to 3.0 ml of (3-mercaptopropyl)trimethoxysilane (MPTMS), and 50 g of toluene were placed in a reactor and then allowed to react at 90°C for 4 hr. The resulting reaction product was washed and then dried under reduced pressure at 70°C for 20 hr, thus obtaining a silica-(SiO₂)-containing microneedle having a sulfhydryl (-SH) functional group (No. 1).

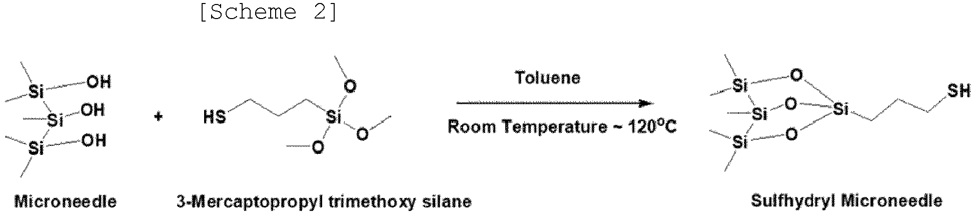

### 1-2-8 to 1-2-10: Surface modification using dealkoxylation

The reaction was carried out under the conditions of Example 1-2-7, with the exception that the reaction time was changed to 8, 16 and 24 hr. After the reaction, the resulting reaction product was dried under reduced pressure at 70°C for 4 hr, thus obtaining silica-(SiO₂)-containing microneedles having sulfhydryl (-SH) functional groups (Nos. 2 to 4) .

### 1-2-11 and 1-2-12: Surface modification using dealkoxylation

The reflux reaction was carried out under the conditions of Example 1-2-7, with the exception that the reaction time was changed to 16 and 24 hr at a pH of 2 to 3. After the reaction, the resulting reaction product was dried under reduced pressure at 70°C for 4 hr, thus obtaining silica-(SiO₂)-containing microneedles having sulfhydryl (-SH) functional groups (Nos. 5 and 6).

The molar number of the sulfhydryl (-SH) functional group per g of the silica-(SiO₂)-containing microneedle having the sulfhydryl (-SH) functional group obtained as described above was measured using a spectrophotometer (Agilent 8453 UV Spectrophotometer) through an Ellman assay. The results thereof are shown in Table 2 below and in FIG. 2.

**[Table 2]**

| No. | Abs. (412) | -SH molar concentration (M) | -SH molar number (mol) | -SH molar number (µmol) | SH molar number for 1 g of MN (µmol) |
|---|---|---|---|---|---|
| 1 | 0.0702 | 4.962E-06 | 1.092E-08 | 0.01092 | 0.9786 |
| 2 | 0.0816 | 5.764E-06 | 1.268E-08 | 0.11268 | 0.9853 |
| 3 | 0.1346 | 9.516E-06 | 2.093E-08 | 0.02093 | 1.791 |
| 4 | 0.1345 | 9.502E-06 | 2.091E-08 | 0.02091 | 1.772 |
| 5 | 0.6658 | 4.706E-05 | 1.035E-07 | 0.1035 | 8.855 |
| 6 | 0.7859 | 5. 554E-05 | 1.222E-07 | 0.1222 | 10.055 |

As is apparent from Table 2 and FIG. 2, the -SH molar number for 1 g of MN was determined to be 0.9786 to 10.055 pmollg, depending on the reaction temperature and the reaction time.

### 1-3: Binding of drug with linker having sulfhydryl (-SH) functional group

### 1-3-1: Binding of peptide drug with linker

The names and abbreviations of the amino acids used in the present invention are set forth below.

### Lys: lysine / His: histidine / Gly: glycine / Ser: serine / Thr: threonine / Arg: arginine / Gln: glutamine / Met: methionine / Glu: glutamic Acid

### 1-3-1-1: Preparation of sulfhydryl-tripeptides represented by Chemical Formula 2 and Chemical Formula 3

As shown in methods (1) to (6) below and in [Scheme 3] below, the peptides were continuously coupled according to the same synthesis cycle. Specifically, the following amino acids in the order of amino acid sequences of compounds 2 and 3 were placed in a reaction vessel and peptide bonding progressed, and finally, a linker X having a sulfhydryl (-SH) functional group [thioglycolic acid (TA), 3-mercaptopropionic acid (MPA), 11-mercaptoundecanoic acid (MUD) and cysteine (CS)] was added thereto and amide bonding progressed, thus preparing sulfhydryl-tripeptides represented by Chemical Formula 2 and Chemical Formula 3.
(1) Washing three times with DMF solvent (10 mL) / washing three times with dichloromethane (DCM) solvent (10 mL)
(2) Deprotection two times for 10 min using 20% (w/v) piperidine DMF solution (3 mL)
(3) Washing five times with DMF solvent (10 mL)
(4) Fmoc-amino acid addition
(5) Amino acid activation and coupling for 2 hr by adding coupling reagent
(6) Washing five times with DMF solvent (10 mL)

[Chemical Formula 2]: Lys-His-Gly-X

[Chemical Formula 3]: Gly-His-Lys-X

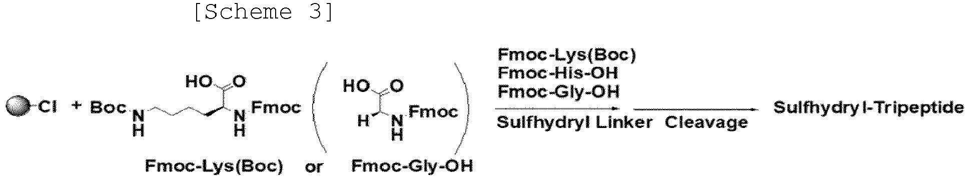

### 1-3-1-2: Preparation of sulfhydryl-pentapeptides represented by Chemical Formula 4 and Chemical Formula 5

Sulfhydryl-pentapeptides represented by Chemical Formula 4 and Chemical Formula 5 were prepared through the peptide synthesis method described in 1-3-1-1 above and as shown in [Scheme 4] below.

[Chemical Formula 4]: Ser-Lys-Thr-Thr-Lys-X

[Chemical Formula 5]: Lys-Thr-Thr-Lys-Ser-X

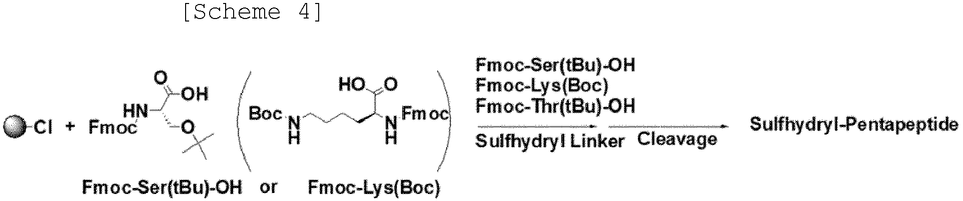

### 1-3-1-3: Preparation of sulfhydryl-hexapeptides represented by Chemical Formula 6 and Chemical Formula 7

Sulfhydryl-hexapeptides represented by Chemical Formula 6 and Chemical Formula 7 were prepared through the peptide synthesis method described in 1-3-1-1 above and as shown in [Scheme 5] below.

[Chemical Formula 6]: Arg-Arg-Gln-Met-Glu-Glu-X

[Chemical Formula 7]: Glu-Glu-Met-Gln-Arg-Arg-X

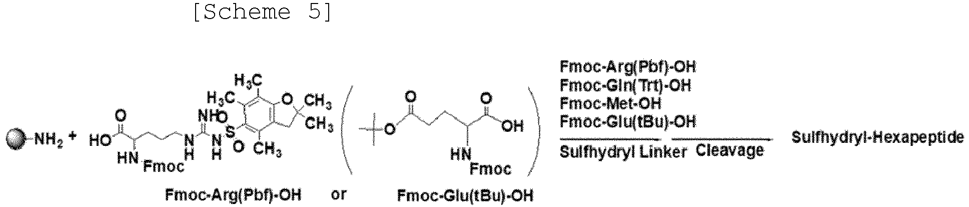

### 1-3-2: Binding of synthetic compound drug with linker

As shown in [Scheme 6] below, dehydroascorbic acid was reacted with a lipase and a linker X having a sulfhydryl (-SH) functional group [thioglycolic acid (TA), 3-mercaptopropionic acid (MPA), 11-mercaptoundecanoic acid (MUD) and cysteine] and reduced, thus preparing a synthetic compound having a sulfhydryl (-SH) functional group, as represented by Chemical Formula 8 below.

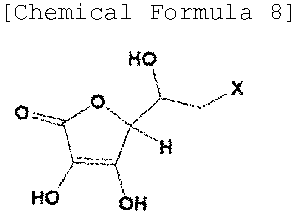

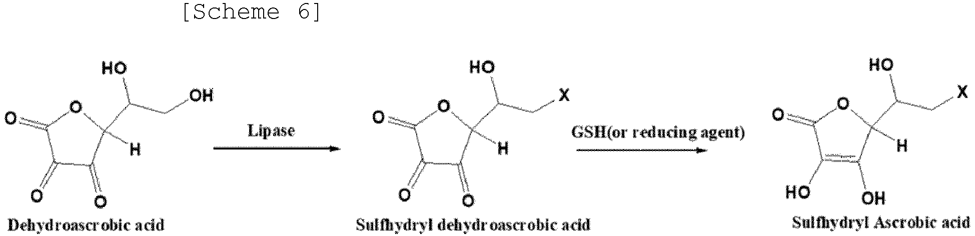

### 1-4: Manufacture of microneedle having drug attached thereto through disulfide bonding

The silica-(SiO₂)-containing microneedle having the sulfhydryl (-SH) functional group manufactured in Example 1-2 and the drug having the sulfhydryl (-SH) functional group manufactured in Example 1-3 were subjected to an oxidation reaction and thus attached to each other through disulfide bonding.

### 1-4-1: Attachment of peptide drug

### 1-4-1-1: Disulfide bonding through oxidation using iodine

0.3 g of the sulfhydryl microneedle manufactured in Example 1-2 and 5.0 mL of purified water/ethanol (50% ethanol) were placed in a reaction vessel and stirred for 60 min so that the microneedle was uniformly dispersed, after which 12.7 µg (0.5 µmol) of iodine (I₂, molar molecular weight: 253.81, Tokyo Chemical Industry Co. Ltd.) was added thereto, 1.2 pmol of the peptide of each of Chemical Formulas 2 to 7 prepared in Example 1-3-1 was added thereto, and the resulting mixture was stirred. After termination of the reaction, washing five times or more with 5.0 mL of ethanol and drying under reduced pressure at 25°C for 12 hr were performed, thus manufacturing a microneedle having a peptide attached thereto through disulfide bonding.

### 1-4-1-2: Disulfide bonding through oxidation using potassium phosphate

A microneedle having a peptide attached thereto through disulfide bonding was manufactured in the same manner as in 1-4-1-1, with the exception that 5.0 mL of purified water was used in lieu of 5.0 mL of purified water/ethanol (50% ethanol) and 40.8 µg (0.3 µmol) of potassium phosphate (KH₂PO₄, molar molecular weight: 136.06 g/mol) was used in lieu of 12.7 µg (0.5 µmol) of iodine (I₂, molar molecular weight: 253.81, Tokyo Chemical Industry Co. Ltd.).

### 1-4-1-3: Disulfide bonding through oxidation using DDQ

A microneedle having a peptide attached thereto through disulfide bonding was manufactured in the same manner as in 1-4-1-1, with the exception that 5.0 mL of dichloromethane was used in lieu of 5.0 mL of purified water/ethanol (50% ethanol) and 68.1 µg (0.3 µmol) of 2,3-dichloro-5,6-dicyano-p-benzoquinone (DDQ, molar molecular weight: 227.0 g/mol) was used in lieu of 12.7 µg (0.5 µmol) of iodine (I₂, molar molecular weight: 253.81, Tokyo Chemical Industry Co. Ltd.).

### 1-4-1-4: Disulfide bonding through oxidation using dehydroascorbic acid

A microneedle having a peptide attached thereto through disulfide bonding was manufactured in the same manner as in 1-4-1-1, with the exception that 5.0 mL of a 0.2 M sodium acetate buffer was used in lieu of 5.0 mL of purified water/ethanol (50% ethanol) and 52.84 µg (0.3 µmol) of dehydroascorbic acid (molar molecular weight: 174.11 g/mol) was used in lieu of 12.7 µg (0.5 µmol) of iodine (I₂, molar molecular weight: 253.81, Tokyo Chemical Industry Co. Ltd.).

### 1-4-1-5: Disulfide bonding through oxidation using hydrogen peroxide

A microneedle having a peptide attached thereto through disulfide bonding was manufactured in the same manner as in 1-4-1-1, with the exception that 5.0 mL of a 0.2 M sodium phosphate buffer was used in lieu of 5.0 mL of purified water/ethanol (50% ethanol) and 10 µL of 30% hydrogen peroxide was used in lieu of 12.7 µg (0.5 µmol) of iodine (I₂, molar molecular weight: 253.81, Tokyo Chemical Industry Co. Ltd.).

### 1-4-2: Attachment of synthetic compound drug

### 1-4-2-1: Disulfide bonding through oxidation using iodine

0.3 g of the sulfhydryl microneedle manufactured in Example 1-2 and 5.0 mL of purified water/ethanol (50% ethanol) were placed in a reaction vessel and stirred for 60 min so that the microneedle was uniformly dispersed, after which 12.7 µg (0.5 µmol) of iodine (I₂, molar molecular weight: 253.81, Tokyo Chemical Industry Co. Ltd.) was added thereto, 112.6 µg (0.45 µmol) of the compound of Chemical Formula 8 prepared in Example 1-3-2 was added thereto, and the resulting mixture was stirred. After termination of the reaction, washing five times or more with 5.0 mL of purified water and ethanol and drying under reduced pressure at 70°C for 12 hr were performed, thus manufacturing a microneedle having ascorbic acid attached thereto through disulfide bonding.

### 1-4-2-2: Disulfide bonding through oxidation using potassium phosphate

A microneedle having ascorbic acid attached thereto through disulfide bonding was manufactured in the same manner as in 1-4-2-1, with the exception that 5.0 mL of purified water was used in lieu of 5.0 mL of purified water/ethanol (50% ethanol) and 40.8 µg (0.3 µmol) of potassium phosphate (KH₂PO₄, molar molecular weight: 136.06 g/mol) was used in lieu of 12.7 µg (0.5 µmol) of iodine (I₂, molar molecular weight: 253.81, Tokyo Chemical Industry Co. Ltd.).

### 1-4-2-3: Disulfide bonding through oxidation using DDQ

A microneedle having ascorbic acid attached thereto through disulfide bonding was manufactured in the same manner as in 1-4-2-1, with the exception that 5.0 mL of dichloromethane was used in lieu of 5.0 mL of purified water/ethanol (50% ethanol) and 68.1 µg (0.3 µmol) of 2,3-dichloro-5,6-dicyano-p-benzoquinone (DDQ, molar molecular weight: 227.0 g/mol) was used in lieu of 12.7 µg (0.5 µmol) of iodine (I₂, molar molecular weight: 253.81, Tokyo Chemical Industry Co. Ltd.).

### 1-4-2-4: Disulfide bonding through oxidation using dehydroascorbic acid

A microneedle having ascorbic acid attached thereto through disulfide bonding was manufactured in the same manner as in 1-4-2-1, with the exception that 5.0 mL of a 0.2 M sodium acetate buffer was used in lieu of 5.0 mL of purified water/ethanol (50% ethanol) and 52.84 µg (0.3 µmol) of dehydroascorbic acid (molar molecular weight: 174.11 g/mol) was used in lieu of 12.7 µg (0.5 µmol) of iodine (I₂, molar molecular weight: 253.81, Tokyo Chemical Industry Co. Ltd.).

### 1-4-2-5: Disulfide bonding through oxidation using hydrogen peroxide

A microneedle having ascorbic acid attached thereto through disulfide bonding was manufactured in the same manner as in 1-4-2-1, with the exception that 5.0 mL of a 0.2 M sodium phosphate buffer was used in lieu of 5.0 mL of purified water/ethanol (50% ethanol) and 10 µL of 30% hydrogen peroxide was used in lieu of 12.7 µg (0.5 µmol) of iodine (I₂, molar molecular weight: 253.81, Tokyo Chemical Industry Co. Ltd.).

### Experimental Example 1: Evaluation of collagen synthesis promotion capability of drug having sulfhydryl (-SH) functional group

### 1-1: Cell line selection and culture

Fibroblasts (CCD-986SK) purchased from the Korea Cell Line Bank were seeded to the bottom of a culture dish, after which DMEM (Dulbecco's Modified Eagle's Medium) containing penicillin (100 IU/mL), streptomycin (100 ug/mL) and 10% fetal bovine serum (FBS) was added thereto, followed by culture in a 5% CO₂ incubator at 37°C.

### 1-2: Cytotoxicity test method (CCK assay)

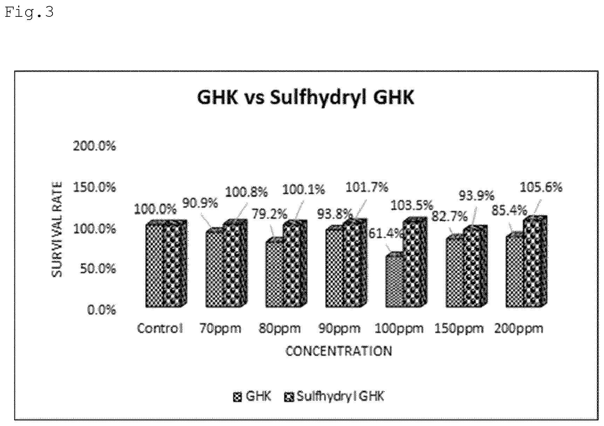

Fibroblasts (CCD-986SK) were aliquoted in an amount of 1.0 × 10⁴ (to 5.0 × 10⁴) cells per well into a 24-well (or 96-well) plate and then cultured for 24 hr under cell culture conditions. The culture medium was discarded, followed by washing with PBS and replacement with new DMEM not containing 10% FBS, after which the cells were treated with a test substance (GHK and GHK-TA) at a predetermined concentration and then cultured for 24 hr. Thereafter, CCK was added to the culture medium in an amount equivalent to 1/10 thereof, followed by measurement at 450 nm using an ELISA reader, and the results thereof are shown in FIG. 3.

As shown in FIG. 3, a GHK peptide exhibited average cell viability of 82.2% at a concentration of 70 to 200 ppm, and GHK-TA exhibited average cell viability of 100.9% at a concentration of 70 to 200 ppm, and was thus confirmed to be stable.

### 1-3: Measurement of collagen content in cell line

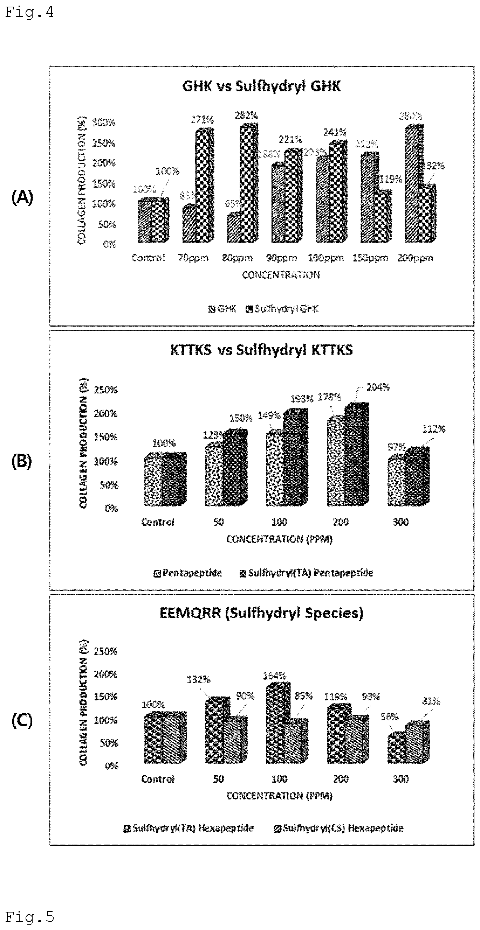

Fibroblasts (CCD-986SK) were aliquoted in an amount of 1.0 × 10⁴ to 5.0 × 10⁴ cells per well into a 24-well (or 96-well) plate and then cultured for 24 hr under cell culture conditions. The culture medium was discarded, followed by washing with PBS, after which the cells were treated with a test substance (Tripeptide (GHK), (GHK)-TA prepared in Example 1-3-1-1, Pentapeptide (KTTKS), (KTTKS)-TA prepared in Example 1-3-1-2, Hexapeptide (EEMQRR), and (EEMQRR)-TA prepared in Example 1-3-1-3) and new DMEM was added thereto, followed by culture for 24 hr. The culture supernatant was collected and the amount of collagen was measured as described below according to the guidelines of the Ministry of Food and Drug Safety, and the results thereof are shown in FIG. 4.

100 µℓ of an antibody-PoD conjugate solution was placed in each well, after which the culture solution and a standard solution were added in an amount of 20 µL thereto and cultured at 37°C for 3 hr. The culture solution was removed from the well, followed by washing four times with 400 µL of a phosphate-buffered saline (PBS). 100 µL of a color development reagent (Procollagen type I peptide EIA kit, Takara Biomedical Co.) was added thereto, followed by culture at room temperature for 15 min, addition with 100 µL of 1 N sulfuric acid, and measurement at 450 nm using an ELISA reader.

As shown in FIG. 4, in the case of GHK, collagen synthesis and promotion of fibroblasts (CCD-986sk) did not occur at a concentration of 80 ppm or less, but sulfhydryl GHK (GHK-TA) exhibited superior collagen synthesis and promotion effects even at a concentration of 70 to 80 ppm. Thus, the peptides of GHK and sulfhydryl GHK (GHK-TA) were composed of the same amino acid sequence, but when the sulfhydryl group was introduced, GHK-TA exhibited the function thereof at a low concentration of 70 to 100 ppm, whereas GHK exhibited efficacy thereof at a relatively high concentration.

In the case of KTTKS, the collagen synthesis and promotion effects increased in proportion to the concentration up to 50 to 200 ppm, and the effects of sulfhydryl KTTKS (KTTKS-TA) were vastly superior compared to KTTKS.

In the case of EEMQRR, it was found that the use of thioglycolic acid (TA) as the linker exhibited superior collagen synthesis and promotion effects compared to the use of cysteine (CS) as the linker.

### Experimental Example 2: Evaluation of collagen synthesis promotion capability of drug-coated microneedle

### 2-1: Collagen production capability depending on linker X having sulfhydryl (-SH) functional group

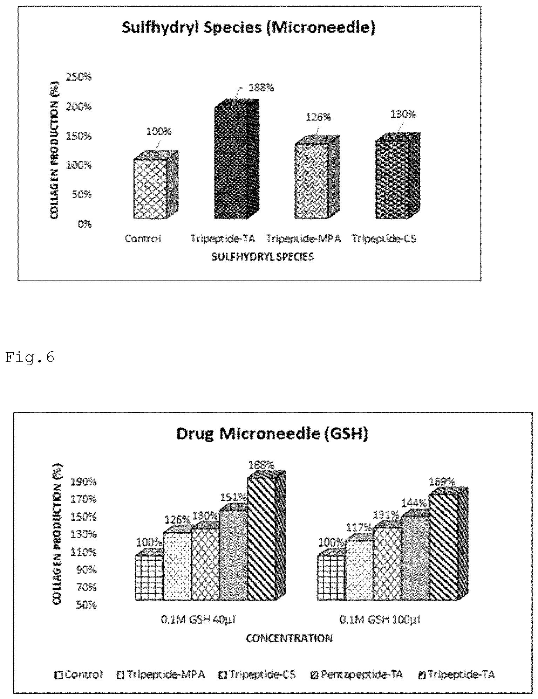

0.3 g of the drug-coated microneedle (GHK-TA-MN, GHK-MPA-MN, GHK-CS-MN) manufactured in Example 1-4 was added with 40 µl of 0.1 M GSH (glutathione) to detach the peptide from the microneedle for 840 min, and the supernatant was then centrifuged, after which intracellular collagen synthesis promotion capability was evaluated in the same manner as in Experimental Example 1-3. The results thereof are shown in FIG. 5.

As shown in FIG. 5, the collagen synthesis promotion capability varied depending on the type of linker X having a sulfhydryl (-SH) functional group. This difference is deemed to be due to the molecular weight of the linker having the sulfhydryl (-SH) functional group. For reference, the molecular weight of the linker is in the order of TA < CS < MPA, and CS has an amine group on the side branch.

### 2-2: Collagen production capability depending on concentration of GSH (glutathione)

0.3 g of the drug-coated microneedle (GHK-MPA-MN, GHK-CS-MN, KTTKS-TA-MN, GHK-TA-MN) manufactured in Example 1-4 was added with 40 µl of 0.1 M GSH (glutathione) to detach the peptide from the microneedle for 840 min, and the supernatant was then centrifuged, after which intracellular collagen synthesis promotion capability was evaluated in the same manner as in Experimental Example 1-3. The results thereof are shown in FIG. 5.

Also, 0.3 g of the drug-coated microneedle (GHK-MPA-MN, GHK-CS-MN, KTTKS-TA-MN, GHK-TA-MN) manufactured in Example 1-4 was added with 100 µl of 0.1 M GSH (glutathione) to detach the peptide from the microneedle for 180 min, and the supernatant was then centrifuged, after which intracellular collagen synthesis promotion capability was evaluated in the same manner as in Experimental Example 1-3. The results thereof are shown in FIG. 6.

As shown in FIG. 6, when using GSH (glutathione) in amounts of 40 µl and 100 ul, the collagen production rates were similar. Therefore, it can be found that, when GSH (glutathione) is present only in the minimum amount necessary to dissociate the disulfide bond, the drug attached to the microneedle can be released, and the effects thereof can be exhibited.

### Preparation Example 1: Cosmetic containing drug-coated microneedle

### 1-1: Preparation of ampoule

A cream was prepared with the composition of Table 3 below.

**[Table 3]**

| NO | Full ingredient list | Amount (wt%) |
|---|---|---|
| 1 | Purified water | Remainder |
| 2 | Glycerin | 2.495 |
| 3 | 1,2-Hexanediol | 2.102 |
| 4 | Methyl propanediol | 2.000 |
| 5 | Propanediol | 2.000 |
| 6 | Butylene glycol | 0.550 |
| 7 | Propylene glycol | 0.505 |
| 8 | *Spongilla* tripeptide-1 | 0.500 |
| 9 | Acetyl hexapeptide-8 | 0.500 |
| 10 | Copper tripeptide-1 | 0.500 |
| 11 | Polysorbate 80 | 0.500 |
| 12 | Allantoin | 0.200 |
| 13 | Panthenol | 0.100 |
| 14 | Trehalose | 0.100 |
| 15 | Sodium hyaluronate | 0.020 |
| 16 | Polyglutamic acid | 0.015 |
| 17 | Caviar extract | 0.015 |
| 18 | Phenethyl alcohol | 0.010 |
| 19 | Hydroxyethyl cellulose | 0.002 |
| 20 | Saccharide isomerate | 0.005 |
| 21 | Methionine | 0.001 |
| 22 | Serine | 0.001 |
| 23 | Histidine | 0.001 |
| 24 | Lecithin | 0.000 |
| 25 | SH-oligopeptide-1 | 0.000 |
| 26 | Polysorbate 20 | 0.000 |
| 27 | Disodium EDTA | 0.030 |
| 28 | Carbomer | 0.450 |
| 29 | Dehydroxanthan gum | 0.050 |
| 30 | Arginine | 0.450 |
| 31 | Fragrance | 0.020 |

### Experimental Example 3: Efficacy of cosmetic containing drug-coated microneedle

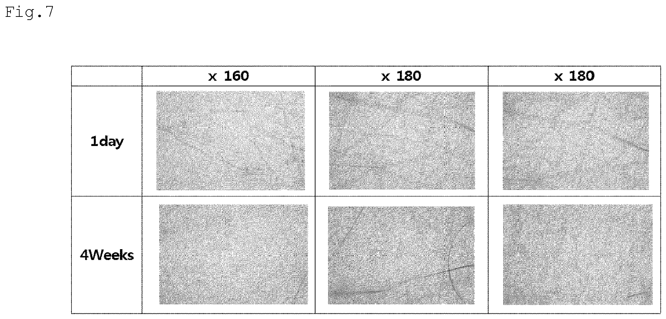

The ampoule prepared in Preparation Example 1 (material name: *Spongilla* tripeptide-1) was gently applied twice a day for 4 weeks (morning and evening) onto the skin in the direction of skin texture in an amount as large as a 100-won coin size, lightly tapped, and allowed to be absorbed into the skin. Skin changes were observed using a microscope on the 2^{nd} week and the 4^{th} week of sample application, and the results thereof are shown in FIG. 7.

As shown in FIG. 7, it was found that the cream containing GHK-TA-MN was excellent in wrinkle reduction effect.

Although specific embodiments of the present invention have been disclosed in detail above, it will be obvious to those skilled in the art that the description is merely of preferable exemplary embodiments and is not to be construed to limit the scope of the present invention. Therefore, the substantial scope of the present invention will be defined by the appended claims.

### Industrial Applicability

According to the present invention, a drug-coated microneedle is capable of effectively delivering a drug having excellent functionality but low skin permeability, and is thus useful as a material for functional cosmetics for whitening, wrinkle reduction, inflammation reduction and the like.

## Claims

1. A method of manufacturing a drug-coated microneedle, which is represented by Chemical Formula 1 below and in which a drug is released through a redox reaction after penetration into a skin, comprising:
(a) manufacturing a silica-(SiO₂)-containing microneedle having a sulfhydryl (-SH) functional group by modifying a surface of a silica-(SiO₂)-containing microneedle;
(b) manufacturing a drug having a sulfhydryl (-SH) functional group by binding a drug with a linker having a sulfhydryl (-SH) functional group; and
(c) subjecting the silica-(SiO₂)-containing microneedle having the sulfhydryl (-SH) functional group and the drug having the sulfhydryl (-SH) functional group to an oxidation reaction:
[Chemical Formula 1] MN-S-S-D
in Chemical Formula 1, MN is a silica-(SiO₂)-containing microneedle, S-S is a disulfide bond, and D is a drug.

2. The method of claim 1, wherein the silica-(SiO₂)-containing microneedle is an acicular spicule derived from a sponge.

3. The method of claim 1, wherein the modifying the surface of the silica-(SiO₂)-containing microneedle is performed by subjecting the silica-(SiO₂)-containing microneedle to stirring with any one selected from the group consisting of (3-mercaptopropyl)methyldimethoxysilane, (3-mercaptopropyl)trimethoxysilane, thioglycolic acid, 3-mercaptopropionic acid, 11-mercaptoundecanoic acid, and 3,3'-dithiodipropionic acid.

4. The method of claim 1, wherein the drug having the sulfhydryl (-SH) functional group is at least one selected from the group consisting of Chemical Formulas 2 to 8 below:
[Chemical Formula 2]: Lys-His-Gly-X
[Chemical Formula 3]: Gly-His-Lys-X
[Chemical Formula 4]: Ser-Lys-Thr-Thr-Lys-X
[Chemical Formula 5]: Lys-Thr-Thr-Lys-Ser-X
[Chemical Formula 6]: Arg-Arg-Gln-Met-Glu-Glu-X
[Chemical Formula 7]: Glu-Glu-Met-Gln-Arg-Arg-X 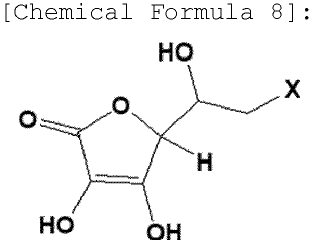
in Chemical Formulas 2 to 8, X is at least one selected from the group consisting of thioglycolic acid, 3-mercaptopropionic acid, 11-mercaptoundecanoic acid, and cysteine.

5. The method of claim 1, wherein the oxidation reaction of the silica-(SiO₂)-containing microneedle having the sulfhydryl (-SH) functional group and the drug having the sulfhydryl (-SH) functional group is performed using at least one oxidant selected from the group consisting of iodine, potassium phosphate, 2,3-dichloro-5, 6-dicyano-p-benzoquinone (DDQ), dehydroascorbic acid, hydrogen peroxide (H₂O₂), and oxygen (O₂).

6. A drug-coated microneedle, which is represented by Chemical Formula 1 below and in which a drug is released through a redox reaction after penetration into a skin:
[Chemical Formula 1] MN-S-S-D
in Chemical Formula 1, MN is a silica-(SiO₂)-containing microneedle, S-S is a disulfide bond, and D is a drug.

7. The drug-coated microneedle of claim 6, wherein the silica- (SiO₂)-containing microneedle is an acicular spicule derived from a sponge.

8. The drug-coated microneedle of claim 6, wherein the microneedle represented by Chemical Formula 1 is manufactured by attaching a silica-(SiO₂)-containing microneedle having a sulfhydryl (-SH) functional group and a drug having a sulfhydryl (-SH) functional group to each other.

9. The drug-coated microneedle of claim 8, wherein the silica-(SiO₂)-containing microneedle includes the sulfhydryl (-SH) functional group in an amount of 0.8 × 10⁻⁶ mol/g to 10 × 10⁻⁶ mol/g.

10. The drug-coated microneedle of claim 8, wherein the drug having the sulfhydryl (-SH) functional group is at least one selected from the group consisting of Chemical Formulas 2 to 8 below:
[Chemical Formula 2]: Lys-His-Gly-X
[Chemical Formula 3]: Gly-His-Lys-X
[Chemical Formula 4]: Ser-Lys-Thr-Thr-Lys-X
[Chemical Formula 5]: Lys-Thr-Thr-Lys-Ser-X
[Chemical Formula 6]: Arg-Arg-Gln-Met-Glu-Glu-X
[Chemical Formula 7]: Glu-Glu-Met-Gln-Arg-Arg-X 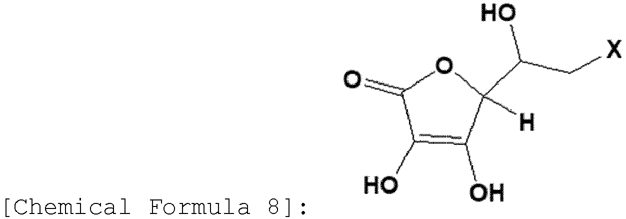
in Chemical Formulas 2 to 8, X is at least one selected from the group consisting of thioglycolic acid, 3-mercaptopropionic acid, 11-mercaptoundecanoic acid, and cysteine.

11. A cosmetic composition comprising the microneedle of any one of claims 6 to 10.

12. The cosmetic composition of claim 11, wherein a cosmetic is selected from the group consisting of a softening lotion, nourishing lotion, nourishing cream, massage cream, essence, ampoule, gel, eye cream, cleansing cream, cleansing foam, cleansing water, pack, spray, and powder.

## Patentansprüche

1. Verfahren zur Herstellung einer arzneimittelbeschichteten Mikronadel, welche durch die nachstehende chemische Formel 1 repräsentiert wird und in der ein Arzneimittel nach dem Eindringen in eine Haut durch eine Redoxreaktion freigesetzt wird, umfassend:
(a) Herstellen einer Siliciumdioxid-(SiO₂)-haltigen Mikronadel mit einer funktionellen Sulfhydryl-(-SH)-Gruppe durch Modifizieren einer Oberfläche einer Siliciumdioxid-(SiO₂)-haltigen Mikronadel;
(b) Herstellen eines Arzneimittels mit einer funktionellen Sulfhydryl-(-SH)-Gruppe durch Verbinden eines Arzneimittels mit einem Linker mit einer funktionellen Sulfhydryl-(-SH)-Gruppe; und
(c) Unterziehen der Siliciumdioxid-(SiO₂)-haltigen Mikronadel mit der funktionellen Sulfhydryl-(-SH)-Gruppe und des Arzneimittels mit der funktionellen Sulfhydryl-(-SH)-Gruppe einer Oxidationsreaktion:
[Chemische Formel 1] MN-S-S-D,
in der chemischen Formel 1 ist MN eine Siliciumdioxid(SiO₂)-haltige Mikronadel, S-S ist eine Disulfidbindung und D ist ein Arzneimittel.

2. Verfahren nach Anspruch 1, wobei die Siliciumdioxid-(SiO₂)-haltige Mikronadel eine azikulare, von einem Schwamm gewonnene Schwammnadel ist

3. Verfahren nach Anspruch 1, wobei das Modifizieren der Oberfläche der Siliciumdioxid-(SiO₂)-haltigen Mikronadel durchgeführt wird, indem die Siliciumdioxid-(SiO₂)-haltige Mikronadel einem Rühren mit einem beliebigen Stoff, ausgewählt aus der Gruppe bestehend aus (3-Mercaptopropyl)methyldimethoxysilan, (3-Mercaptopropyl)trimethoxysilan, Thioglykolsäure, 3-Mercaptopropionsäure, 11-Mercaptoundecansäure und 3,3'-Dithiodipropionsäure, unterzogen wird.

4. Verfahren nach Anspruch 1, wobei das Arzneimittel mit der funktionellen Sulfhydrylgruppe (-SH) mindestens eines ist ausgewählt aus der Gruppe bestehend aus den nachstehenden chemischen Formeln 2 bis 8:
[Chemische Formel 2]: Lys-His-Gly-X
[Chemische Formel 3]: Gly-His-Lys-X
[Chemische Formel 4]: Ser-Lys-Thr-Thr-Lys-X
[Chemische Formel 5]: Lys-Thr-Thr-Lys-Ser-X
[Chemische Formel 6]: Arg-Arg-Gln-Met-Glu-Glu-X
[Chemische Formel 7]: Glu-Glu-Met-Gln-Arg-Arg-X 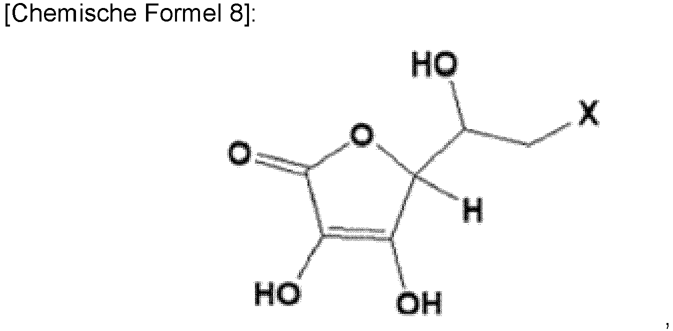
in den chemischen Formeln 2 bis 8 ist X mindestens eines ausgewählt aus der Gruppe bestehend aus Thioglykolsäure, 3-Mercaptopropionsäure, 11-Mercaptoundecansäure und Cystein.

5. Verfahren nach Anspruch 1, wobei die Oxidationsreaktion der Siliciumdioxid-(SiO₂)-haltigen Mikronadel mit der Sulfhydryl-(-SH)-Funktionsgruppe und des Arzneimittels mit der Sulfhydryl-(-SH)-Funktionsgruppe unter Verwendung mindestens eines Oxidationsmittels, ausgewählt aus der Gruppe bestehend aus Iod, Kaliumphosphat, 2,3-Dichlor-5,6-dicyano-p-benzochinon (DDQ), Dehydroascorbinsäure, Wasserstoffperoxid (H₂O₂) und Sauerstoff (O₂), durchgeführt wird.

6. Eine arzneimittelbeschichteten Mikronadel, welche durch die nachstehende chemische Formel 1 repräsentiert wird und in der ein Arzneimittel nach dem Eindringen in eine Haut durch eine Redoxreaktion freigesetzt wird:
[Chemische Formel 1] MN-S-S-D,
in der chemischen Formel 1 ist MN eine Siliciumdioxid(SiO₂)-haltige Mikronadel, S-S ist eine Disulfidbindung und D ist ein Arzneimittel.

7. Mit einem Arzneimittel beschichtete Mikronadel nach Anspruch 6, wobei die Siliciumdioxid-(SiO₂)-haltige Mikronadel eine azikulare, von einem Schwamm gewonnene Schwammnadel ist

8. Die arzneimittelbeschichtete Mikronadel nach Anspruch 6, wobei die durch die chemische Formel 1 repräsentierte Mikronadel hergestellt wird, indem eine Siliciumdioxid-(SiO₂)-haltige Mikronadel mit einer funktionellen Sulfhydryl-(-SH)-Gruppe und ein Arzneimittel mit einer funktionellen Sulfhydryl-(-SH)-Gruppe aneinander gehängt werden.

9. Arzneimittel-beschichtete Mikronadel nach Anspruch 8, wobei die Siliciumdioxid-(SiO₂)-haltige Mikronadel die funktionelle Sulfhydryl-(-SH)-Gruppe in einer Menge von 0,8 × 10⁻⁶ mol/g bis 10 × 10⁻⁶ mol/g umfasst.

10. Medikamentenbeschichtete Mikronadel nach Anspruch 8, wobei das Medikament mit der funktionellen Sulfhydryl-(-SH)-Gruppe mindestens eines ist ausgewählt aus der Gruppe bestehend aus den nachstehenden chemischen Formeln 2 bis 8:
[Chemische Formel 2]: Lys-His-Gly-X
[Chemische Formel 3]: Gly-His-Lys-X
[Chemische Formel 4]: Ser-Lys-Thr-Thr-Lys-X
[Chemische Formel 5]: Lys-Thr-Thr-Lys-Ser-X
[Chemische Formel 6]: Arg-Arg-Gln-Met-Glu-Glu-X
[Chemische Formel 7]: Glu-Glu-Met-Gln-Arg-Arg-X 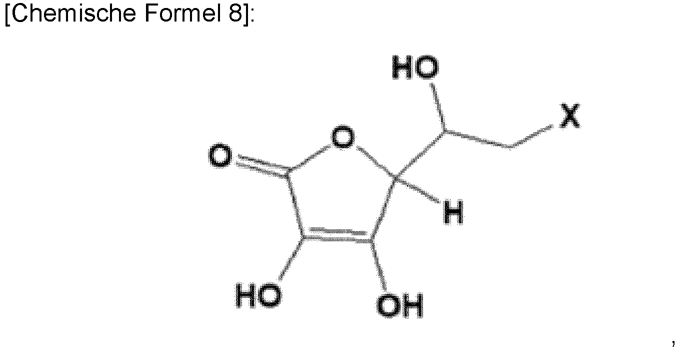
in den chemischen Formeln 2 bis 8 ist X mindestens eines ausgewählt aus der Gruppe bestehend aus Thioglykolsäure, 3-Mercaptopropionsäure, 11-Mercaptoundecansäure und Cystein.

11. Kosmetische Zusammensetzung, umfassend die Mikronadel nach einem der Ansprüche 6 bis 10.

12. Kosmetische Zusammensetzung nach Anspruch 11, wobei ein Kosmetikum ausgewählt ist aus der Gruppe bestehend aus einer weichmachenden Lotion, pflegenden Lotion, pflegenden Creme, Massagecreme, Essenz, Ampulle, Gel, Augencreme, Reinigungscreme, Reinigungsschaum, Reinigungswasser, Packung, Spray und Puder.

## Revendications

1. Procédé de fabrication d'une micro-aiguille revêtue de médicament, qui est représentée par la formule chimique 1 ci-dessous et dans laquelle un médicament est libéré par l'intermédiaire d'une réaction redox après pénétration dans la peau, comprenant :
(a) la fabrication d'une micro-aiguille contenant de la silice (SiO₂) ayant un groupe fonctionnel sulfhydryle (-SH) par modification de la surface d'une micro-aiguille contenant de la silice (SiO₂) ;
(b) la fabrication d'un médicament ayant un groupe fonctionnel sulfhydryle (-SH) par liaison d'un médicament avec un lieur ayant un groupe fonctionnel sulfhydryle (-SH) ; et
(c) la soumission de la micro-aiguille contenant de la silice (SiO₂) ayant le groupe fonctionnel sulfhydryle (-SH) et du médicament ayant le groupe fonctionnel sulfhydryle (-SH) à une réaction d'oxydation :
[formule chimique 1] MN-S-S-D
dans la formule chimique 1, MN est une microaiguille contenant de la silice (SiO₂), S-S est une liaison disulfure, et D est un médicament.

2. Procédé selon la revendication 1, dans lequel la micro-aiguille contenant de la silice (SiO₂) est un spicule aciculaire dérivé d'une éponge.

3. Procédé selon la revendication 1, dans lequel la modification de la surface de la micro-aiguille contenant de la silice (SiO₂) est effectuée par soumission de la micro-aiguille contenant de la silice (SiO₂) à une agitation avec l'un quelconque choisi dans le groupe constitué par le (3-mercaptopropyl)méthyldiméthoxysilane, le (3-mercaptopropyl)triméthoxysilane, l'acide thioglycolique, l'acide 3-mercaptopropionique, l'acide 11-mercaptoundécanoïque, et l'acide 3,3'-dithiodipropionique.

4. Procédé selon la revendication 1, dans lequel le médicament ayant le groupe fonctionnel sulfhydryle (-SH) est au moins l'un choisi dans l'ensemble constitué par les formules chimiques 2 à 8 ci-dessous :
[formule chimique 2] : Lys-His-Gly-X
[formule chimique 3] : Gly-His-Lys-X
[formule chimique 4] : Ser-Lys-Thr-Thr-Lys-X
[formule chimique 5] : Lys-Thr-Thr-Lys-Ser-X
[formule chimique 6] : Arg-Arg-Gln-Met-Glu-Glu-X
[formule chimique 7] : Glu-Glu-Met-Gln-Arg-Arg-X 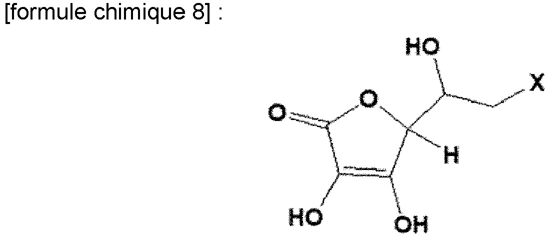
dans les formules chimiques 2 à 8, X est au moins l'un choisi dans l'ensemble constitué par l'acide thioglycolique, l'acide 3-mercaptopropionique, l'acide 11-mercaptoundécanoïque, et la cystéine.

5. Procédé selon la revendication 1, dans lequel la réaction d'oxydation de la micro-aiguille contenant de la silice (SiO₂) ayant un groupe fonctionnel sulfhydryle (-SH) et du médicament ayant un groupe fonctionnel sulfhydryle (-SH) est effectuée par utilisation d'au moins un oxydant choisi dans l'ensemble constitué par l'iode, le phosphate de potassium, la 2,3-dichloro-5,6-dicyano-p-benzoquinone (DDQ), l'acide déshydroascorbique, le peroxyde d'hydrogène (H₂O₂) et l'oxygène (O₂).

6. Micro-aiguille revêtue de médicament, qui est représentée par la formule chimique 1 ci-dessous et dans laquelle un médicament est libéré par l'intermédiaire d'une réaction redox après pénétration dans la peau :
[formule chimique 1] MN-S-S-D
dans la formule chimique 1, MN est une microaiguille contenant de la silice (SiO₂), S-S est une liaison disulfure, et D est un médicament.

7. Micro-aiguille revêtue de médicament selon la revendication 6, dans laquelle la micro-aiguille contenant de la silice (SiO₂) est un spicule aciculaire dérivé d'une éponge.

8. Micro-aiguille revêtue de médicament selon la revendication 6, dans laquelle la micro-aiguille représentée par la formule chimique 1 est fabriquée par attachement l'un à l'autre d'une micro-aiguille contenant de la silice (SiO₂) ayant un groupe fonctionnel sulfhydryle (-SH) et d'un médicament ayant un groupe fonctionnel sulfhydryle (-SH).

9. Micro-aiguille revêtue de médicament selon la revendication 8, dans laquelle la micro-aiguille contenant de la silice (SiO₂) contient le groupe fonctionnel sulfhydryle (-SH) en une quantité de 0,8 × 10⁻⁶ mol/g à 10 × 10⁻⁶ mol/g.

10. Micro-aiguille revêtue de médicament selon la revendication 8, dans laquelle le médicament ayant un groupe fonctionnel sulfhydryle (-SH) est au moins l'un choisi dans l'ensemble constitué par les formules chimiques 2 à 8 ci-dessous :
[formule chimique 2] : Lys-His-Gly-X
[formule chimique 3] : Gly-His-Lys-X
[formule chimique 4] : Ser-Lys-Thr-Thr-Lys-X
[formule chimique 5] : Lys-Thr-Thr-Lys-Ser-X
[formule chimique 6] : Arg-Arg-Gln-Met-Glu-Glu-X
[formule chimique 7] : Glu-Glu-Met-Gln-Arg-Arg-X 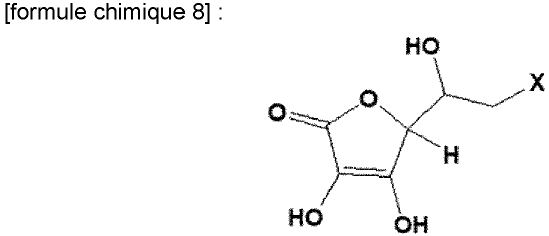
dans les formules chimiques 2 à 8, X est au moins l'un choisi dans l'ensemble constitué par l'acide thioglycolique, l'acide 3-mercaptopropionique, l'acide 11-mercaptoundécanoïque, et la cystéine.

11. Composition cosmétique comprenant la micro-aiguille de l'une quelconque des revendications 6 à 10.

12. Composition cosmétique selon la revendication 11, dans laquelle le cosmétique est choisi dans l'ensemble constitué par une lotion adoucissante, une lotion nourrissante, une crème nourrissante, une crème de massage, une essence, une ampoule, un gel, une crème pour les yeux, une crème nettoyante, une mousse nettoyante, une eau nettoyante, un pack, un spray, et une poudre.
